# EUROPEAN PATENT SPECIFICATION

(11) **EP 1 834 598 B1**
(45) Date of publication and mention of the grant of the patent: **22.07.2009**
(21) Application number: 07011802.1
(22) Date of filing: 24.05.2006
(51) Int. Cl.: A61B 18/00

(54) **High frequency treatment tool**
Hochfrequenzbehandlungsgerät
Outil de traitement des hautes fréquences

(30) Priority: 25.05.2005 JP 2005152005; 25.05.2005 JP 2005152007
(43) Date of publication of application: 19.09.2007
(62) Divisional of application: 06010717.4
(73) Proprietor: Fujinon Corporation, Saitama-shi, Saitama (JP)
(72) Inventor: Akiba, Haruo, Saitama (JP); Machiya, Mamoru, Saitama (JP); Ooyatsu, Masayuki, Saitama (JP)
(74) Representative: Klunker . Schmitt-Nilson . Hirsch

(56) References cited:
- DE-A1- 10 312 485
- DE-A1- 19 602 759
- JP-A- 8 299 355
- US-A1- 2004 210 284
- US-B1- 6 203 533

## Description

### Background of the Invention

### 1. Field of the Invention

The present invention relates to a high frequency treatment tool that is inserted into a treatment tool insertion channel of an endoscope and used for performing a treatment such as incision of a diseased mucous membrane.

### 2. Description of the Related Art

When a diseased portion such as a tumor is found on the mucous membrane on a body cavity inner wall of the gullet, stomach, duodenum, or colon by endoscopic inspection, a treatment is performed to excise the portion of the diseased mucous membrane by using a high frequency treatment tool. In this case, to secure safety of the treatment, the treatment is performed under observation through an endoscope, and the high frequency treatment tool used for the treatment is inserted into the treatment tool insertion channel of the endoscope and guided to the portion to be treated. Herein, on the body cavity inner wall, the submucosal layer exists below the mucosal layer, and the muscle layer is covered by the submucosal layer. The treatment to incise and remove the diseased mucosal layer by using the high frequency treatment tool must be performed so as not to leave the diseased portion and so as not to damage the muscle layer at all.

The high frequency treatment tool to be used for incising the mucosal layer is formed by attaching a high frequency knife formed of an electrode member having a rod-like portion inside a flexible sheath, and to the base end of the flexible sheath, operating means is joined, and by a remote operation on this operating means, the high frequency knife can be controlled to stick out and withdraw into the tip end of the flexible sheath. By supplying a current to the high frequency knife sticking out from the flexible sheath, the mucous membrane can be cauterized and incised.

As a structure of the electrode member forming the high frequency knife, there are available a needle-like knife formed by extending a rod-like electrode member straight, and a hook knife having a hook portion formed by continuously providing a large diameter electrode portion on the tip end of the rod-like electrode member or bending the tip end of the electrode member into almost an L shape. The needle-like knife is operated so as to stab the mucous membrane, and can incise the mucous membrane by horizontally moving or swinging the electrode member. On the other hand, the hook knife catches the tissue of the mucous membrane by the hook portion on the tip end and is operated so as to be drawn to the inserting portion side of the endoscope to incise the mucous membrane.

As described above, during current supply to the high frequency knife, the high frequency knife must be reliably maintained in a state without contact with the muscle layer. When the needle-like knife is used, the needle-like knife is positioned ahead of the flexible sheath and punctures the mucous membrane, so that in some cases of performing the treatment, the tip end of the needle-like knife cannot be captured in the observation field of the endoscope. Therefore, unless the sticking-out length and sticking-out direction of the needle-like knife from the flexible sheath are accurately controlled, the safety of the treatment cannot be secured.

On the other hand, the hook knife is caught on the mucous membrane under observation through the endoscope, and next, the hook knife is drawn into the treatment tool insertion channel while supplied with a high frequency current, whereby incising the mucous membrane. Therefore, during operations of the hook knife, the tip end of the hook knife can always be operated under observation through the endoscope, so that it can be operated so as not to come into contact with the muscle layer when it is supplied with a current.

However, when using the hook knife, to smoothly catch the tissue of the mucous membrane, the tip end of the hook knife must be stable. Therefore, a high frequency treatment tool having a mechanism for stabilizing the hook knife during actuation is proposed in JP-A-2004-313537 whose priority is claimed by US 2004/0210 284. In the high frequency treatment tool of this JP-A-2004-313537, an electrical insulating member is attached to the tip end of the flexible sheath, a through hole is formed in this electrical insulating member, the rod-like portion of the electrode member forming the hook knife is inserted into the through hole, and the hook portion on the tip end can come into contact with and separate from the tip end outer surface of the electrical insulating member. When it is supplied with current, the electrode member is made to stick out by a predetermined length from the flexible sheath, and the diameter difference between the hole diameter of the through hole and the outer diameter of the electrode member is minimized and the sticking-out length of the electrode member is restricted, whereby stably retaining the electrode member.

During the treatment to excise the diseased portion by using the above-described electrode member, bleeding occurs in some cases, and this may make it impossible to confirm the diseased portion. Therefore, in the electrical insulating member, an opening separate from the through hole for inserting the electrode member is formed, or the through hole is formed into a cross shape or a triangular shape, whereby forming a liquid flow-out portion that the rod-like portion of the electrode member cannot enter is formed. A syringe is connected to the base end of the flexible sheath and filled with normal saline solution, and by operating this syringe, the normal saline solution can be jetted to the bleeding portion from the liquid flow-out portion to wash the portion.

When the high frequency knife is a hook knife, the mucous membrane or submucosal layer is caught by this hook knife and the hook knife is operated so as to be drawn into the treatment tool insertion channel, and in this state, the tissue is cut by supplying a current to the hook knife, and then the hook knife is led out again from the treatment tool insertion channel, and these operations are repeated. Therefore, efficiency and quickness of the operations cannot be obtained, and it takes a long time to remove the diseased mucous membrane, and accordingly, the pain of the examinee and the burden on the operator increase.

On the other hand, when a needle-like knife is used, the efficiency and quickness of operations of a treatment can be obtained. However, when a needle-like knife is used, to secure safety and reliability of the treatment, the needle-like knife must be disposed inside the flexible sheath except in the case where the treatment is performed by using the needle-like knife, and the sticking-out length of the needle-like knife from the flexible sheath must be restricted when the treatment is performed. To prevent the tip end of the needle-like knife from damaging the healthy tissues, when the diseased mucous membrane is excised, the needle-like knife must be operated so as not to come into contact with the muscle layer positioned below the mucous membrane.

In addition, the hook portion of the high frequency knife is positioned forward of the electrical insulating member provided on the tip end of the flexible sheath and always exposed to the outside. Therefore, for example, during the operation of insertion into the treatment tool insertion channel, if the electrode member is supplied with a current by mistake, it damages the channel inner wall. In addition, when a liquid is jetted, the hook portion is positioned in front of the jetting passage, so that the jetted liquid is obstructed by the hook portion and it becomes impossible to accurately jet the liquid toward a target portion.

### Summary of the Invention

The invention was developed in view of the above-described circumstances, and an object thereof is to provide a high frequency treatment tool in which the high frequency knife is not exposed to the outside and can be safely operated, and which can efficiently feed a liquid to a desired position.

To achieve the object, according to the invention, a high frequency treatment tool which can be inserted into a body cavity via a treatment tool insertion channel of an endoscope, the high frequency treatment tool comprises: a flexible sheath; a treatment tool main body comprising a flexible cord and a straight electrode member on a tip end of the flexible cord, the treatment tool main bodybeing attached inside the flexible sheath, and a high frequency current capable of being applied to the straight electrode member; a partition member comprising an insertion hole which the electrode member is stuck out from and withdrawn into, the partition member being fixedly attached inside the flexible sheath in such a manner that an tip face of the partition member is provided at almost the same position as a tip end face of the flexible sheath; and a stopper member that restricts a sticking-out length of the electrode member from the insertion hole, the stopper member being formed in the treatment tool main body so as to come into contact with and separate from a base end side surface of the partition member, wherein at least one liquid jetting passage for jetting a liquid supplied from inside of the flexible sheath is formed in the partition member, and a liquid feed passage is formed in the stopper member and the liquid feed passage communicates with the liquid j ettingpassage at an arbitrary rotating position of the stopper member.

As the electrode member, a straight one, that is, a needle-like knife is used. This needle-like knife is inserted into the flexible sheath, and the electrode member is reliably housed within the flexible sheath at times other than actual treatment by using the high frequency treatment tool. When a treatment is performed, the electrode member is made to stick out, however, the sticking-out length of this electrode member is restricted. The flexible sheath is provided with a partition member, the treatment tool main body side including the electrode member is provided with a stopper member, and this stopper member prevents the electrode member from sticking out over a position at which the stopper member comes into contact with the partition member. Herein, the partition member and the stopper member are both made of a hard material, and therefore, to smoothly insert the high frequency treatment tool into the treatment tool insertion channel of an endoscope, in particular, it is not allowed to excessively lengthen the length in the axial direction of the partition member fixedly provided in the flexible sheath. The high frequency treatment tool is a long flexible member, and by winding the high frequency treatment tool into a loop or inserting it into a treatment tool insertion channel while the endoscope inserting portion is bent, the flexible sheath and the treatment tool main body relatively deviate from each other, and the electrode member is displaced closer to the base end side than the partition member having the insertion hole. Namely, the electrode member comes out inward from the insertion hole. Therefore, when a treatment is performed, the electrode member must be operated so as to enter the insertion hole. When the electrode member is made to stick out from the par tit ion member, if the diameter difference between the inner diameter of the insertion hole and the outer diameter of the electrode member is reduced to stably retain the electrode member and if the electrode member deviates from the insertion hole, entering into the insertion hole becomes impossible. By providing a draw-in portion on the partition member, the electrode member can be guided into the insertion hole, however, to more reliably insert the electrode member into the insertion hole, the diameter difference between the outer diameter of the stopper member and the inner diameter of the flexible sheath is reduced, and inside the flexible sheath, movements of the electrode member other than in the axial direction are restricted. Therefore, the stopper member substantially moves so as to slide with respect to the inner wall of the flexible sheath. As a result, the electrode member is retained almost at the axial center position of the flexible sheath, so that it is reliably guided into the insertion hole.

For example, during a treatment, if the mucous membrane bleeds, the bleeding portion is washed away by jetting a liquid such as normal saline solution. For this, the inside of the flexible sheath is used as a path for feeding the liquid. Namely, inside the flexible sheath, the clearance between the same and the flexible cord of the treatment tool main body is used as a path for feeding the liquid. Inside the flexible sheath, a stopper member is attached near the tip end of the flexible cord, and a partition member is fixedly provided at the tip end of the flexible sheath. In the partition member, a liquid jetting passage is formed, and a liquid feed passage is formed in the stopper member. The partition member must be provided with a contact area necessary for firm fixation to the inner surface of the flexible sheath. Therefore, the size of the communicating passage is limited. On the other hand, at the base end side of the partition member, as a stopper member is provided, and to stabilize the posture of the electrode member, the outer diameter of the stopper member is made large, however, this stopper member is not necessarily made to contact by a large area with the flexible sheath.

From the description given above, the liquid jetting passage to be formed in the partition member can be a groove or a through hole. Herein, when it is a groove, it is formed on the outer circumferential side. The number of liquid jetting passages may be one, and preferably plural, for example, three. The liquid feed passages to be formed in the stopper member can also be formed by grooves or through holes. When the liquid feed passages are formed by through holes, their radial positions overlap the liquid jetting grooves of the partition member; and their pitches in the circumferential direction are set smaller than the widths in the circumferential direction of the liquid jetting passages. The hole diameters of the through holes are desirably formed as large as possible. The liquid feed passage can also be formed by grooves formed in the outer circumference of the stopper member. Herein, the stopper member is only required to stabilize the position in the axial direction of the electrode member inside the flexible sheath, so that it is not necessary to make the sliding area on the flexible sheath very large. Therefore, the depths of the grooves forming the liquid feed passages are set almost the same as those of the grooves of the partition member, and the widths in the circumferential direction are set larger than the widths in the circumferential direction between the grooves of the partition member. The numbers of grooves to be formed in the partition member and the stopper member can be the same, however, they can be di f ferent , for example, the number of grooves on the partition member side is three and the number of grooves on the stopper member side is four. Accordingly, it is preferable that the liquid jetting passage comprises a plurality of grooves or through holes provided at an outer circumferences of the partition member, and the liquid feed passage comprises a plurality of grooves or through holes provided at an outer circumferences of the stopper member.

By employing the above-described construction, a treatment such as incision of the mucous membrane can be safely performed by using the needle-like knife, and the treatment can be smoothly, reliably, and efficiently performed, and a liquid can be accurately jetted to a bleeding portion or the like.

Further, effects can be obtained in that the high frequency knife is prevented from being exposed to the outside and can be safely operated when treatment is not performed with the high frequency treatment tool, and a fluid such as nofrmal saline solution can be efficiently supplied as appropriate.

### Brief Description of the Drawings

Fig. 1 is an entire construction view of a high frequency treatment tool of an embodiment of the invention;
Fig. 2 is a main part enlarged sectional view of Fig. 1;
Fig. 3 is an enlarged sectional view of the tip end of the treatment tool main body, showing a state in that the needle-like knife moves to the position of the first guide collar;
Fig. 4 is a sectional view similar to Fig. 3, showing an actuating state in that the needle-like knife passes through the position of the first guide collar and advances to the position of the second guide collar from the state of Fig. 3;
Fig. 5 is a sectional view similar to Fig. 3, showing an actuating state in that the needle-like knife is led out from the insertion hole of the second guide collar;
Fig. 6 is a sectional view on X-X of Fig. 4;
Fig. 7 is an external view of a state in that the high frequency treatment tool of an embodiment of the invention is led out from the treatment tool insertion channel of an endoscope;
Fig. 8 is an action explanatory view showing a state of incision by using the high frequency treatment tool;
Fig. 9 is a main part enlarged sectional view of Fig. 1;
Fig. 10 is an enlarged sectional view of the tip end of the treatment tool main body;
Fig. 11 is a sectional view similar to Fig. 10, showing a state in that the electrode member sticks out;
Figs. 12A and 12B are explanatory views showing the positional relationship between the liquid jetting passage grooves provided in the partition member and the liquid feed passage grooves provided in the stopper member; and
Fig. 13 is a front view showing a modified example of the liquid feed passages provided in the stopper member;

### Detailed Description of the Invention

### [First Embodiment]

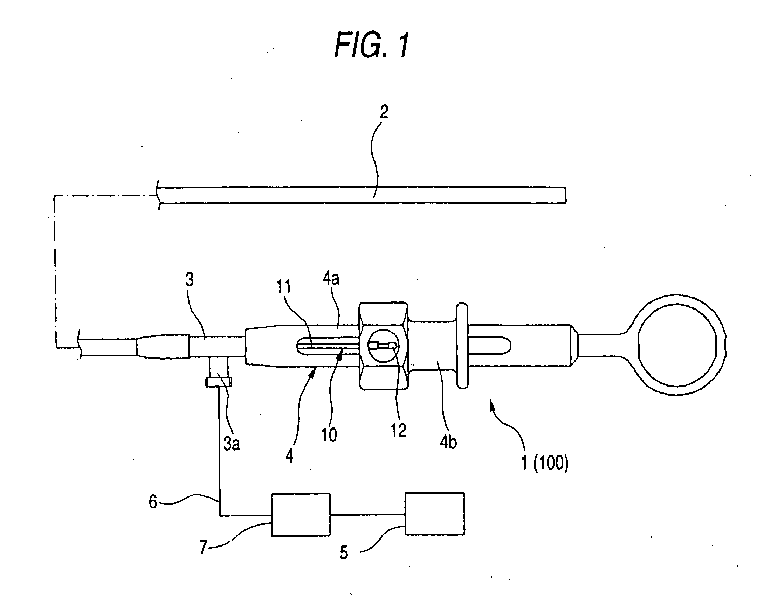

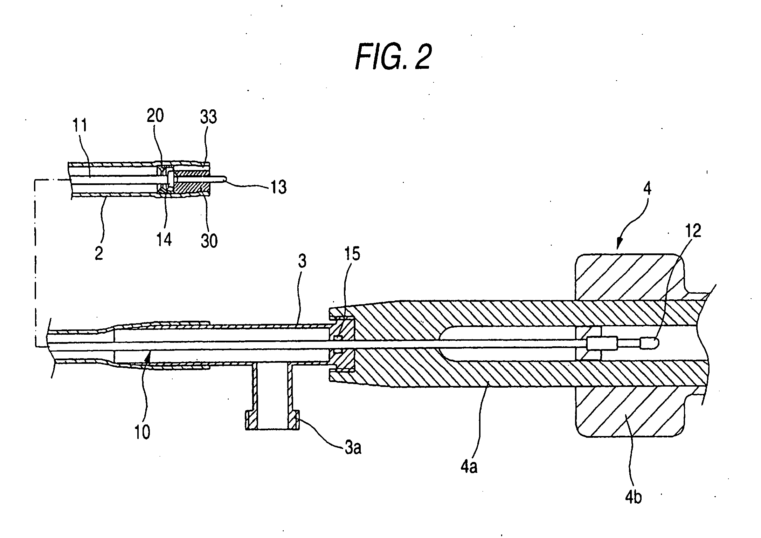

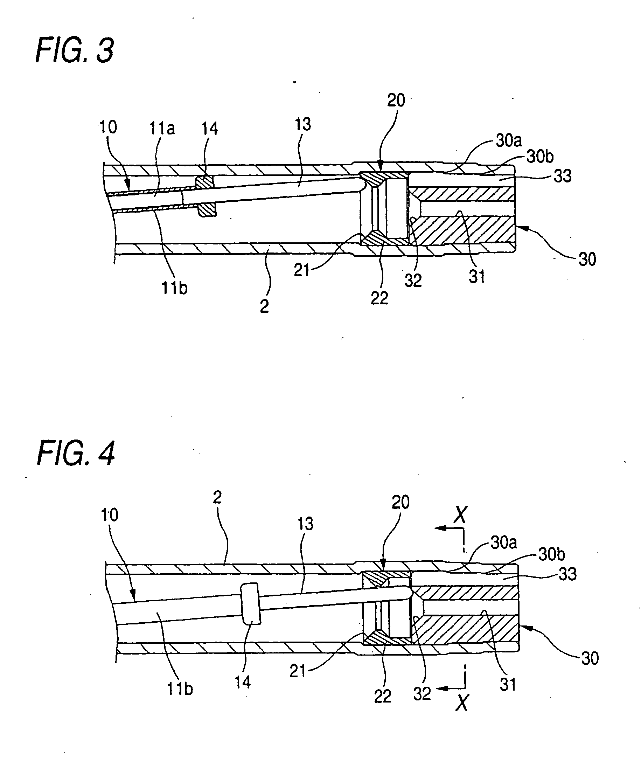

Hereinafter, a first embodiment showing only partly features of the invention will be explained with reference to the drawings. First, Fig. 1 shows an entire construction of a high frequency treatment tool, and Fig. 2 shows a main part enlarged section of the same. Furthermore, Fig. 3 through Fig. 5 show a section of the tip end of the high frequency treatment tool in different actuating states.

First, in Fig. 1 and Fig. 2, the reference numeral 1 denotes a high frequency treatment tool, and this high frequency treatment tool 1 has a long flexible sheath 2, a connecting pipe 3 is joined to the base end of the flexible sheath 2, and operating section 4 is joined to the other end of this connecting pipe 3. The operating section 4 includes a main body shaft 4a joined to the connecting pipe 3 and a slider 4b that is fitted to the main body shaft 4a and is slidable in the axial direction of the main body shaft 4a. To the slider 4b, the base end of a flexible cord 11 forming the treatment tool main body 10 is joined.

The flexible cord 11 is formed by, as clearly seen in Fig. 3, inserting the outer circumference of, for example, a lead wire 11a into an insulating coating 11b, and has flexibility in at least a bending direction. The base end of the lead wire 11a in this flexible cord 11 sticks out by a predetermined length from the portion joined to the slider 4b to form a contact portion 12. This contact portion 12 is connected to an unillustrated high frequency power supply apparatus in a disconnectable manner.

The flexible cord 11 of the treatment tool main body 10 passes through the inside of the connecting pipe 3 from the portion attached to the slider 4b, and extended to the inside of the flexible sheath 2. From the tip end of the flexible cord 11, a needle-like knife 13 is provided in a manner enabling it to stick out. The needle-like knife 13 is preferably formed of a hard rod-like member and electrically connected to the lead wire 11a of the flexible cord 11, and a predetermined length thereof is exposed to the outside, and this portion acts on the internal body tissue when it is supplied with a current to cauterize the tissue, whereby treatment such as incision and exfoliation of the mucous membrane is performed.

The reference numeral 20 denotes a first guide collar, and 30 denotes a second guide collar. Between these first and second guide collars 20 and 30, at least the second guide collar 30 is made of an electrical insulating material, in particular, ceramic. On the other hand, the first guide collar 20 is not necessarily made of an electrical insulating material, however, it can be made of an electrical insulating material such as ceramic or a synthetic resin, or can be made of a conductive material of metal or the like. The first guide collar 20 has an outer diameter slightly larger than the inner diameter of the flexible sheath 2, and is attached inside the flexible sheath 2, and the second guide collar 30 is positioned closer to the tip end side than the first guide collar 20 inside the flexible sheath 2. The tip end face of the second guide collar 30 is disposed at almost the same position as the tip end face of the flexible sheath 2.

On the first guide collar 20, a first guide surface 21 is formed. This first guide surface 21 is formed of a tapered surface inclined diagonally inward at a predetermined angle from the base end side to the tip end side of the flexible sheath 2, and this first guide surface 21 is formed into an annular shape having a predetermined width from the outer circumference to the inner side of the first guide collar 20. At the tip end side of the portion where the first guide surface 21 is formed, a spacer 22 is continuously provided. This spacer 22 is a thin ring-shaped member having an outer diameter equal to that of the outer circumference of the first guide collar 20 and larger than the inner circumferential edge of the first guide surface 21.

In the second guide collar 30, at a position of the central axis line of the flexible sheath 2, an insertion hole 31 is provided. The needle-like knife 13 provided on the tip end of the flexible cord 11 can be inserted into and extracted from this insertion hole 31. Therefore, the inner diameter of the insertion hole 31 is larger than the outer diameter of the needle-like knife 13, and the diameter difference between these is made small so as not to leave a gap, substantially, and the sticking-out length of the needle-like knife 13 from the insertion hole 31 is set to several millimeters or less, for example, 1 through 3 millimeters. As a result, when the needle-like knife 13 is stuck out from the insertion hole 31, this needle-like knife 13 is stably retained in a straight advancing state, and is not bent or deformed by action of an external force or the like. Furthermore, the length in the axial direction of the needle-like knife 13 in the second guide collar 30 is an important element for stabilizing the needle-like knife 13 sticking out from the insertion hole 31. Therefore, the length of the insertion hole 31 is set to a length necessary for stabilizing the needle-like knife 13. Furthermore, on the end face of the second guide collar 30 facing the first guide collar 20, a second guide surface 32 that guides the needle-like knife 13 toward the insertion hole 31 is formed.

It is desirable that the outer circumference of the second guide surface 32 is extended to the further outer side than the inner circumferential edge of the first guide surface 21 when viewed from the axial direction of the flexible sheath 2 to reliably shrift the tip end of the needle-like knife 13 from the first guide surface 21 of the first guide collar 20 to the second guide collar 30. However, as illustrated, the tip end of the needle-like knife 13 is formed into an almost semispherical surface, so that a gap equal to or less than the radius of the needle-like knife 13 is allowed between the inner circumferential edge of the first guide surface 21 and the outer circumference of the second guide surface 32.

In the treatment tool main body 10 including the flexible cord 11 and the needle-like knife 13, at the shifting portion between the needle-like knife 13 and the flexible cord 11, a stopper member 14 is provided. The stopper member 14 is formed of a ring-shaped member with a diameter larger than the outer diameter of the needle-like knife 13 and the inner diameter of the insertion hole 31, and is fixed to the tip end of the insulating coating 11b at the outer circumference of the needle-like knife 13. Preferably, the stopper member 14 can come into contact with and separate from a flat portion shifting from the outer circumference of the second guide surface 32 in the second guide collar 30. The outer diameter of the stopper member 14 is set so as to pass through the inner circumferential edge of the first guide surface 21 of the first guide collar 20.

The stopper member 14 restricts the sticking-out length of the needle-like knife 13 from the tip end face (forming almost the same surface as the second guide surface 30) of the flexible sheath 2, and the maximum sticking-out length is set as described later to a length equal to or more than a thickness of the mucous layer and equal to or less than a total thickness of the mucous layer and the submucosal layer when the tip end face of the flexible sheath 2 is made to contact the mucous membrane surface. Therefore, depending on a portion to which a treatment is applied with the high frequency treatment tool 1, the preferable sticking-out length of the needle-like knife 13 changes.

Furthermore, this high frequency treatment tool 1 has supply section for a liquid such as normal saline solution. This fluid supply section has a pipe connecting portion 3a provided on the connecting pipe 3, and to this pipe connecting portion 3a, a liquid feed pipe 6 from a liquid tank 5 is detachably connected. At the middle of the liquid feed pipe 6, switching section 7 for opening and closing the flow passage like a foot switch is provided to control supply of normal saline solution. Therefore, an annular gap formed between the inside of the flexible sheath 2 joined to the connecting pipe 3, that is, the inner surface of the flexible sheath 2 and the outer surface of the treatment tool main body 10 inserted inside the flexible sheath 2 functions as a liquid feed passage. The flexible cord 11 in the treatment tool main body 10 passes through the connecting pipe 3 and is inserted into the main body shaft 4a, and inside the connecting pipe 3, a seal member 15 is attached around the flexible cord 11 to prevent the fluid from leaking to the operating section 4 side.

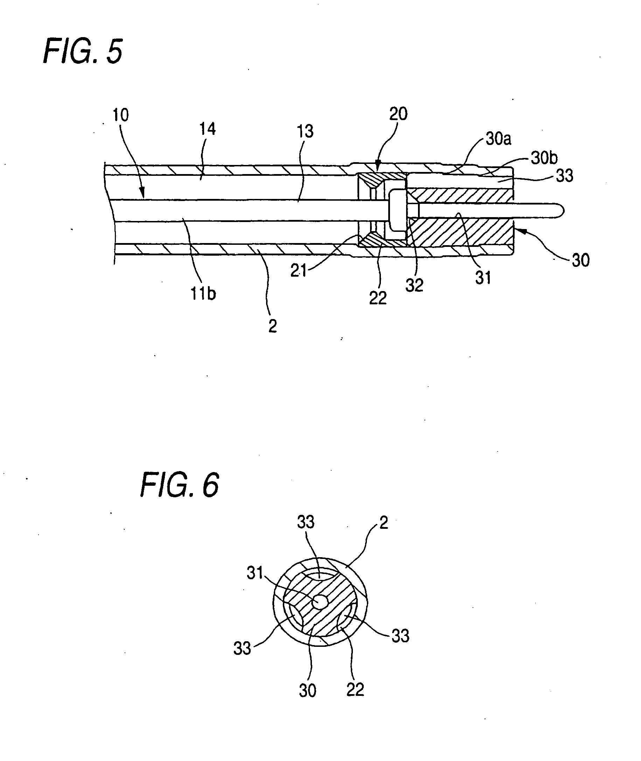

The tip end of the fluid supply passage is opened at the joined portion between the flexible sheath 2 and the second guide collar 30. Namely, on the outer circumferential surface of the second guide collar 30, grooves 33 across the entire length of the axial direction are provided. The grooves 33 are formed at one or several points in the circumferential direction of the second guide collar 30, for example, as shown in Fig. 6, at three points at equal intervals circumferentially. The depths of the grooves 33 reach the further inner side than the inner circumferential surface of the spacer 22 formed in the first guide collar 20, and the further inner side portions than the spacer 22 of the grooves 33 are always opened inside the flexible sheath 2. However, by extending the groove bottoms of the grooves 33 to almost equal to the inner circumferential edge of the first guide surface 21, or further inward by a dimension corresponding to the radius of the needle-like knife 13 than the inner circumferential edge of the first guide surface 21, the section area of the fluid supply passage can be increased.

Furthermore, the first and second guide collars 20 and 30 must be retained so as not to come off the flexible sheath 2. Particularly, when the needle-like knife 13 is made to stick out from the insertion hole 31, the tip end of the needle-like knife 13 is pressed against the guide collars 20 and 30, so that the guide collars 20 and 30 are pushed, however, the first and second guide collars 20 and 30 are stably retained. For retaining these, the outer diameter of the first guide collar 20 is slightly larger than the inner diameter of the flexible sheath 2, and the first guide collar 20 is attached so as to expand the flexible sheath 2. The outer circumferential surface of the second guide collar 30 is formed so that the base end side has the largest diameter, the tip end side has the smallest diameter, and the middle portion has a middle outer diameter, and thereby, vertically stepped portions 30a and 30b are formed on the outer surface of the second guide collar 30. The outer diameter of the smallest diameter portion in this second guide collar 30 is equal to or larger than the diameter of the inner circumferential surface of the flexible sheath 2 in at least a free state. To more firmly fix the first and second guide collars 20 and 30 by the flexible sheath 2, it is also possible that these are fixed by using an adhesive. Furthermore, it is also allowed that screw portions are formed on the outer circumferential surfaces of the guide collars 20 and 30 and screwed to the flexible sheath 2.

By constructing as described above, when the needle-like knife 13 is operated, a force is applied in a direction of pushing the first guide collar 20 and the second guide collar 30 toward the tip end of the flexible sheath 2, however, to these first and second guide collars 20 and 30, a fastening force of the flexible sheath 2 is applied, and the stepped portions 30a and 30b bite into the inner surface of the flexible sheath 2, and as a result, the second guide collar 30 is reliably prevented from coming off.

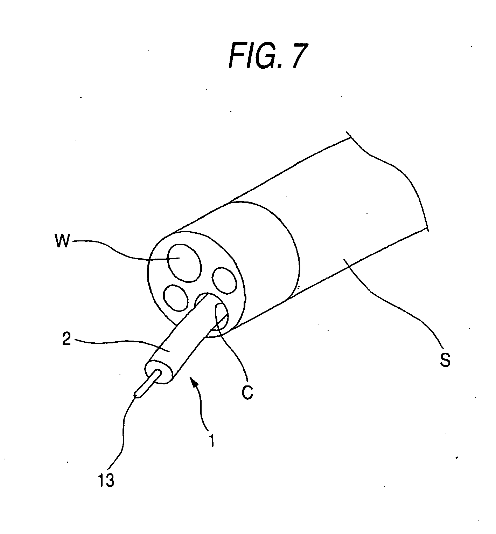

The high frequency treatment tool 1 in this embodiment is constructed as described above, and as shown in Fig. 7, when a diseased mucous membrane exists on a body cavity inner wall of, for example, the gullet, the stomach, the duodenum, or the colon, the high frequency treatment tool is inserted via a treatment tool insertion channel C provided in an endoscope inserting portion S having an observation portion W into the body cavity and used for applying treatment such as incision and exfoliation to remove the diseased mucous membrane. Herein, by observation via the observation portion W of the endoscope inserting portion S, when a diseased portion is found on the mucous membrane, the mucous membrane in a predetermined region including this diseased portion is exfoliated and removed, and as a stage before this, preferably, local injection into the diseased mucous membrane is performed to bulge the mucous membrane.

The high frequency treatment tool 1 is inserted through the treatment tool insertion channel C, and before starting treatment, the needle-like knife 13 of the treatment tool main body 10 is drawn to the inner deep portion of the flexible sheath 2, at least, a position closer to the base end side than the first guide collar 20, preferably, sufficiently further inward of the base end side. Thereby, even when relative position deviation of the treatment tool main body 10 occurs inside the flexible sheath 2 due to insertion of the high frequency treatment tool 1 into the inside of the treatment tool insertion channel C while winding the high frequency treatment tool 1 in a loop form and bending the endoscope inserting portion S, the needle-like knife 13 is reliably positioned inside the flexible sheath 2 and retained so as not to be exposed to the outside. Therefore, even when the high frequency power supply is turned on by mistake and a high frequency current is supplied to the needle-like knife 13, the knife does not come into contact with another object, so that safety is maintained.

When the tip end of the high frequency treatment tool 1 is led out from the treatment tool insertion channel C, the operating section 4 of the high frequency treatment tool 1 is operated to make the needle-like knife 13 to stick out from the tip end of the flexible sheath 2. At this time, when the needle-like knife 13 deviates most from the central axis line of the flexible sheath 2, the tip end of the needle-like knife 13 comes into contact with the inner surface of the flexible sheath 2. In this state, when the operating section 4 is operated to lead-out the needle-like knife 13 from the tip end of the treatment tool insertion channel C, the tip end of the needle-like knife 13 slides along the inner surface of the flexible sheath 2 and comes into contact with the first guide surface 21 of the first guide collar 20 as shown in Fig. 3. The first guide surface 21 is inclined inwardly toward the front side, so that the tip end of the needle-like knife 13 slides on the first guide surface 21 and is reliably moved toward the center of the flexible sheath 2. The first guide surface 21 is formed into an annular tapered surface toward the inner side from the inner surface of the flexible sheath 2, so that the needle-like knife smoothly slides and moves on the first guide surface 21.

When the tip end of the needle-like knife 13 passes through the inner circumferential edge of the first guide surface 21 of the first guide collar 20, it approaches the second guide collar 30. Herein, on the second guide collar 30, a second guide surface 32 is formed, and the second guide surface 32 overlaps the first guide surface 21 or a gap equal to or less than the radius of the needle-like knife 13 is formed between these, so that the tip end of the needle-like knife 13 is reliably guided by the second guide surface 32 and drawn into the insertion hole 31 as shown in Fig. 4.

The first guide surface 21 in the first guide collar 20 has no deficiency on the entire circumference, however, the second guide collar 30 has the grooves 33, so that the needle-like knife 13 must not enter the grooves 33. However, the grooves 33 are positioned closer to the outer circumferential side than the inner side of the first guide surface 21, so that the tip end of the needle-like knife 13 does not enter and are not locked in the grooves 33. Thus, only by pushing the needle-like knife 13 by the operating section 4, the needle-like knife 13 can be guided by the first and second guide surfaces 21 and 32 from the inner circumferential surface of the flexible sheath 2 toward the inside of the insertion hole 31 and reliably guided to the insertion hole 31. Therefore, the diameter difference between the inner diameter of the insertion hole 31 and the outer diameter of the needle-like knife 13 can be minimized so that a gap is not created between these, whereby the led-out portion of the needle-like knife 13 from the flexible sheath 2 can be stably retained and prevented from bending and deforming.

The needle-like knife 13 advances, as shown in Fig. 5, until the stopper member 14 provided on it comes into contact with a portion around the insertion hole 31, more specifically, a flat surface further outward than the second guide surface 32, and the needle-like knife does not stick out more than this. The sticking-out length of the needle-like knife 13 at this point is set so as not to reach the muscle layer positioned below the submucosal layer although it penetrates the mucosal layer when the tip end faces of the first flexible sheath 2 and the second guide collar 30 of the high frequency treatment tool 1 are made to contact the mucous membrane surface.

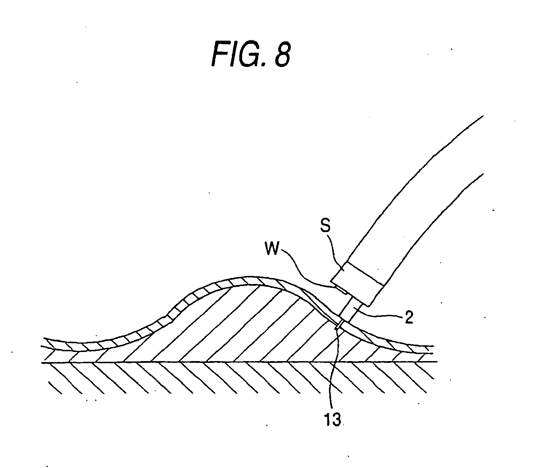

Therefore, as shown in Fig. 8, normal saline solution or the like is locally injected in advance into the submucosal layer so that the submucosal layer is bulged to greatly space the mucosal layer from the muscle layer, and while the tip end of the flexible sheath 2 and the tip end face of the second guide collar 30 provided inside the flexible sheath 2 are made to contact the mucosal layer, the needle-like knife 13 of the treatment tool main body 10 is led out from the flexible sheath 2 as described above to puncture the mucosal layer. The needle-like knife 13 is inserted into the insertion hole 31 in the second guide collar 30 with almost no gap, so that even when the needle-like knife 13 is thin, it is not broken or bent. The needle-like knife 13 punctures the inside of the mucosal layer, and a high frequency current is supplied, whereby the mucosal layer is cauterized, and the needle-like knife 13 does not damage the muscle layer even when supplied with a current as long as the tip end face of the flexible sheath 2 is in contact with the mucous membrane surface, and the mucosal layer can be reliably incised.

After incising the mucosal layer as described above, to exfoliate the mucosal layer from the muscle layer, fibers of the submucosal layer are cut with the needle-like knife 13 sticking-out from the tip end of the flexible sheath 2 to exfoliate the mucous membrane. Then, when incising and exfoliating the mucosal layer, if bleeding occurs, a liquid such as normal saline solution is jetted to the bleeding portion from the liquid feed pipe 6 connected to the pipe connecting portion 3a to wash the bleeding portion. As a result, the field of observation through the observation part W of the endoscope insertion portion 2 becomes excellent.

The mucous membrane including the diseased portion is bulged by the normal saline solution, and during incision, the normal saline solution flows out or is absorbed by the body, so that the bulged portion contracts. Therefore, to maintain the bulged state of the submucosal layer, exfoliation of the mucous membrane can be performed while replenishing the normal saline solution via the same path as described above. Namely, while the needle-like knife 13 is drawn into the insertion hole 31 made in the second guide collar 30 and the tip end face of the flexible sheath 2 is made to contact the submucosal layer, the normal saline solution is supplied by making the pressure inside the flexible sheath 2 high from the pipe connecting portion 3a of the connecting pipe 3, whereby the liquid can be directly fed to the submucosal layer. As a result, the submucosal layer to be exfoliated can be maintained in a bulged state. In addition, by making the tip end face contact the submucosal layer and supplying the normal saline solution toward a necessary portion, the submucosal layer can be reliably maintained in the bulged state, and exfoliation of the mucosal membrane can be safely performed with the needle-like knife 13.

### [Second Embodiment]

Hereinafter, an embodiment of the invention denoted as second embodiment will be described with reference to the drawings. A high frequency treatment tool 100 of the second embodiment of the invention has the same entire construction as in the first embodiment of the invention (See Fig. 1.).

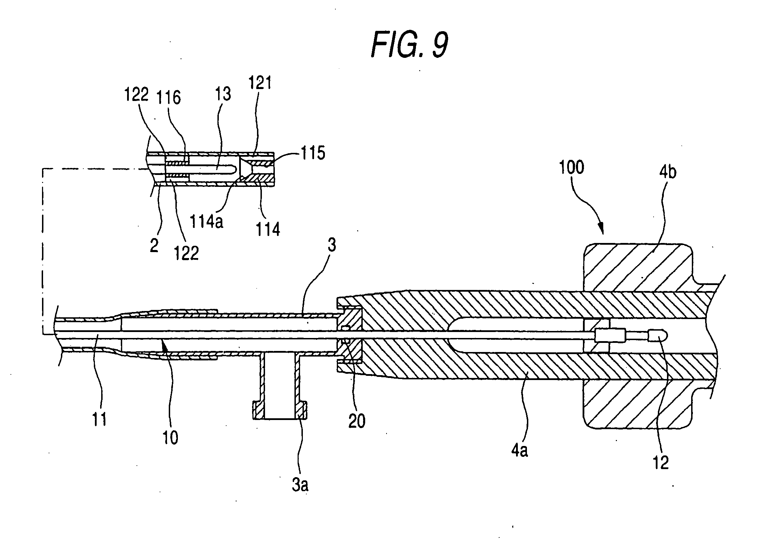

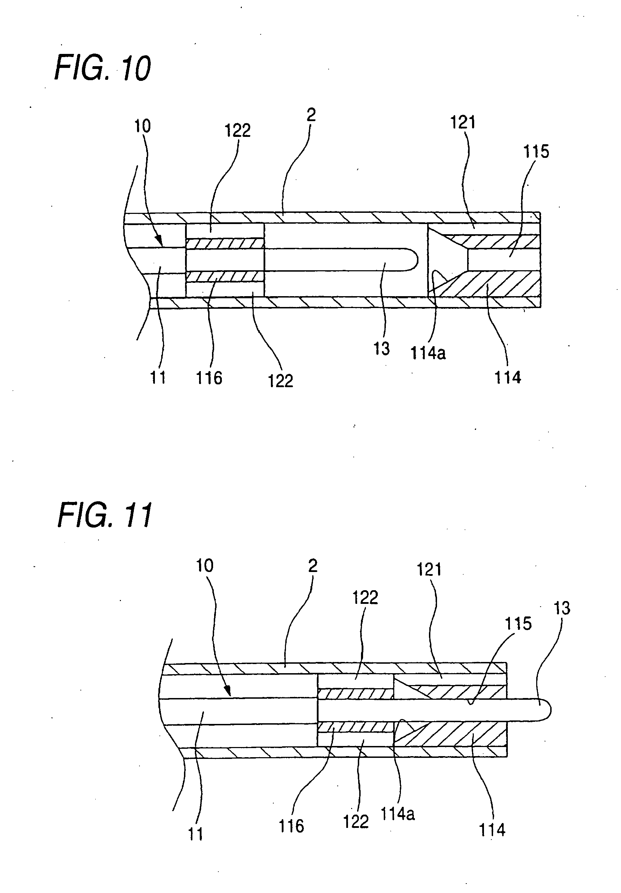

As clearly seen in Fig. 9 (showing a main part enlarged section of Fig. 1), the flexible cord 11 of the treatment tool main body 10 is extended to the inside of the flexible sheath 2 from the connecting portion to the slider 4b through the inside of the connecting pipe 3. From the tip end of the flexible cord 11, a lead wire is extended straight, and the led-out portion of this lead wire forms an electrode member 13 forming a needle-like knife. A partition member 114 is inserted in and fitted to the tip end of the flexible sheath 2 and fixed by section of bonding or the like. The partition member 114 is made of ceramic or the like, and is fixed at a position forming the same surface as the tip end face of the flexible sheath 2 as seen in Fig. 10 and Fig. 11. In the partition member 114, at the position of the central axis line, a through hole 115 is made so as to perforate in the axial direction, and the hole diameter of this insertion hole 115 is set slightly larger than the outer diameter of the electrode member 13. On the base end of the partition member 114, a draw-in tapered portion 114a for guiding the electrode member 13 into the insertion hole 115 is formed.

Furthermore, at the shift portion from the flexible cord 11 to the electrode member 13 in the treatment tool main body 10 or the portion of the electrode member 13, a stopper member 116 is attached. The stopper member 116 has an outer diameter slightly smaller than the inner diameter of the flexible sheath 2, and therefore, when the treatment tool main body 10 is moved inside the flexible sheath 2, it almost slides on the inner surface of the flexible sheath 2. When the electrode member 13 on the tip end of the treatment tool main body 10 sticks out by a predetermined length from the tip end face of the partition member 114, the stopper member 116 comes into contact with the partition member 114 and restricts the electrode member 13 from sticking out more.

By thus making the stopper member 116 contact the partition member 114, a predetermined treatment can be performed while the electrode member 13 sticks out by a predetermined length from the tip end of the flexible sheath 2. Therefore, the sticking-out length of the electrode member 13 at this time depends on the thickness of the tissue to be treated. For example, when incising the mucous membrane, the sticking-out length is set longer than the thickness of the mucosal layer in the body cavity inner wall and shorter than a total thickness of the mucosal layer and the submucosal layer. Thereby, the electrode member 13 punctures the mucous membrane while supplied with a current in a state in that the tip end face of the flexible sheath 2 is made to contact the mucosal layer, the electrode member 13 penetrates the mucosal layer and reaches the submucosal layer, however, it does not reach the muscle layer, so that the treatment can be performed so that the mucosal layer is reliably incised without damage to the muscle layer.

With this high frequency treatment tool 100, a treatment such as incision of the mucosal layer and exfoliation from the muscle layer can be performed, and during this treatment, if bleeding occurs, a liquid such as normal saline solution can be supplied to wash the bleeding portion away. For this, as seen in Fig. 1, the connecting pipe 3 has a connection port 3a, and a liquid feed pipe 6 is connected to this connection port 3a from a liquid tank 5 in a disconnectable manner, and to this liquid feed pipe 6, switching section 7 including a foot switch or the like is attached, and liquid supply is controlled by this switching section 7. Therefore, the inside of the flexible sheath 2 passing through the inside of the connecting pipe 3 from the connection port 3a and connected to the connecting pipe 3 becomes a liquid feed passage. Therefore, the flexible cord 11 is led to the outside via a seal member 20 at the base end of the connecting pipe 3.

At the middle of this liquid feedpassage, thepartitionmember 114 and the stopper member 116 are interposed. The partition member 114 is fixed to the inner surface of the flexible sheath 2, and the stopper member 116 is in frictional contact with the inner surface of the flexible sheath 2. Therefore, a liquid can be jetted from the tip end of the flexible sheath 2 via the partition member 114 and the stopper member 116.

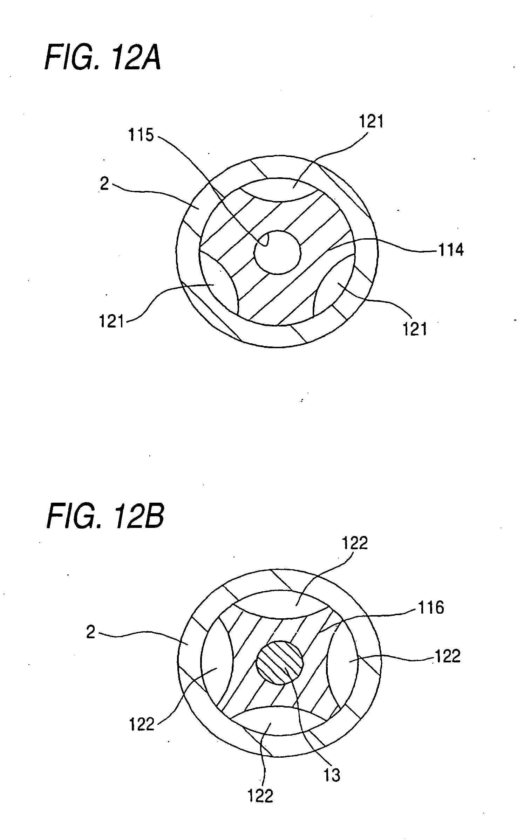

For this, as shown in Fig. 12A, in the outer circumferential surface of the partition member 114, three liquid jetting passage grooves 121 are formed at equal intervals in the circumferential direction. These liquid jetting passage grooves 121 penetrate the partition member 114 in the axial direction. The partition member. 114 must be fixed to the inner surface of the flexible sheath 2. The partition member 114 is inserted inside the flexible sheath 2 and fixed by using an adhesive. The circumferential widths of the liquid jetting passage grooves 121 formed in the outer circumferential surface of the partition member 114 are formed as large as possible in a range that does not deteriorate the fixing performance of the partition member 114 to the flexible sheath 2, whereby increasing the flow area.

Thus, the partition member 114 is fixed to the flexible sheath 2, however, the position in the rotation direction of the stopper member 116 provided on the treatment tool main body 1 side is not restricted on the inner surface of the flexible sheath 2. In the outer circumferential surface of the stopper member 116, as shown in Fig. 12B, liquid feed passage grooves 122 the number of which is more than the liquid jetting passage grooves 121 of the partition member 114, in detail, four liquid feed passage grooves 122 are formed at equal intervals in the circumferential direction. The depths of the liquid feed passage grooves 122 are set almost the same or deeper than the depths of the liquid jetting passage grooves 121, and their lengths in the circumferential direction are set greater than the interval between the liquid jetting passage grooves 121 adjacent to each other in the partition member 114. Thereby, at least a part of the liquid feed passage grooves 122 in the stopper member 116 communicates with the liquid jetting passage grooves 121 at an arbitrary rotating position. By thus constructing the liquid feed passage grooves 122, the interval between the grooves becomes narrow, however, the stopper member 116 is only required to stably retain the electrode member 13 almost at the axial center position of the flexible sheath 2, so that the frictional contact portion with the inner surface of the flexible sheath 2 is allowed to be short.

Thereby, when the electrode member 13 is made to stick out to the maximum sticking-out position and the stopper member 16 comes into contact with the partition member 114, the liquid feed passage grooves 122 partially overlap the liquid jetting passage grooves 121 of the partition member 114 and allow a liquid to be jetted from the tip end of the flexible sheath 2.

A state in that the electrode member 13 is drawn to the inside of the flexible sheath 2 is shown in Fig. 10, and a state in that the electrode member 13 sticks out most from the partition member 114 is shown in Fig. 11. As clearly seen in these drawings, when the electrode member 13 is drawn to the inside of the flexible sheath 2, the tip end of the electrode member 13 is disposed closer to the base end side than the base end face of the partition member 114. On the other hand, in the maximum sticking-out state of the electrode member 13, it is inserted into the insertion hole 115 of the partition member 114 and sticks out by a predetermined length. The pushing and pulling operations of the electrode member 13 are performed by remote operations on the operating section 4.

As shown in Fig. 7, the high frequency treatment tool 100 constructed as described above is inserted into a body cavity via a treatment tool insertion channel C provided in the endoscope inserting portion S having an observing portion W, and when a diseased mucous membrane exists on the body cavity inner wall of, for example, the gullet, the stomach, the duodenum, or the colon, the high frequency treatment tool is used for performing a treatment to exfoliate and remove this diseased mucosal portion. First, by injecting normal saline solution or the like into the diseased mucosal portion by using a syringe, the submucosal layer is evaginated and bulged. Then, by inserting the high frequency treatment tool 1 into the treatment tool insertion channel C, the mucous membrane is incised with this high frequency treatment tool 1. At this point, the liquid feed pipe 6 is connected in advance to the connection port 3a of the connecting pipe 3, and a liquid such as normal saline solution is supplied through the liquid feed pipe 6.

The tip end face of the flexible sheath 2 is made to correctly face the mucosal layer to be excised and lightly pressed against the mucous membrane surface. Herein, the tip end face of the flexible sheath 2 forms almost the same surface as the tip end face of the partition member 114, so that its wide area comes into contact with the mucous membrane surface so that the flexible sheath is stably retained so as not to press the mucous membrane.

In this state, by operating the operating section 4, the electrode member 13 is made to stick out from the tip end face of the flexible sheath 2. Then, by supplying a high frequency current to the electrode member 13, the internal body tissue is cauterized and incised. Herein, the electrode member 13 is positioned closer to the base end side than the insertion hole 115 of the partition member 114, however, near the tip end of the electrode member 13, the stopper member 116 is attached, and this stopper member 116 is almost in contact with the inner circumferential surface of the flexible sheath 2. Therefore, inside the flexible sheath 2, the electrode member 13 is retained in a posture almost matching with the center of axis. Therefore, the electrode member 13 is disposed on almost the same axis as that of the insertion hole 115 formed in the partition member 114, and a draw-in tapered portion 114a is formed around the insertion hole 115 in the partition member 114, so that the electrode member 13 is smoothly and reliably inserted into the insertion hole 115 by a remote operation on the operating section 4.

When the electrode member 13 sticks out most, that is, when the stopper member 116 attached to the electrode member 13 advances to the position in contact with the partition member 114, the tip end of the electrode member 13 penetrates the mucosal layer and reaches the submucosal layer, and punctures to a position that does not reach the muscle layer. Then, while supplying a high frequency current to the electrode member 13, the electrode member 13 is moved along the outer circumference of the diseased mucous membrane by a bending operation or the like of the endoscope inserting portion S. Thereby, the outer circumference of the diseased mucous membrane is incised, and at this time, the muscle layer positioned in the submucosal layer is not damaged at all. Thereafter, fibers of the submucosal layer are cut by action of the electrode member 13, whereby the mucous membrane is exfoliated. The mucosal layer thus exfoliated can be collected with, for example, grasping forceps or the like.

By the above-described operations, the diseased mucous membrane can be completely excised without a remainder, and in addition, the healthy mucous membrane and muscle layer are not damaged at all. The electrode member 13 forms a needle-like knife, and by horizontally moving it along the mucosal layer or by swinging the electrode member 13, the mucous membrane can be excised quickly and efficiently. As a result, the pain of the examinee and the burden on the operator can be reduced.

During the treatment described above, bleeding occurs in some cases. In such a case as bleeding, a liquid is jetted to wash the bleeding portion away, whereby securing an observation field of the observing portion W. For this, a liquid such as normal saline solution is pressure-fed to the inside of the flexible sheath 2 from the liquid feed pipe 6 connected to the connection port 3a of the connecting pipe 3. Thereby, the normal saline solution or the like is jetted to the bleeding portion from the inside of the flexible sheath 2 via the liquid feed passage grooves 122 of the stopper member 116 and the liquid jetting passage grooves 121 of the partition member 114, and the bleeding portion is smoothly washed away. In addition, in some cases, the liquid such as normal saline solution flows out from the submucosal layer bulged by local injection or is absorbed into the body, the bulged portion contracts. Therefore, as described above, it is also possible to replenish the normal saline solution or the like from the liquid jetting passage grooves 121 provided in the partition member 114.

Herein, washing of the bleeding portion and replenishment of normal saline solution can be performed with the electrode member 13 sticking out, however, by drawing the electrode member 13 into the insertion hole 115, the tip end of the flexible sheath 2 can be made to contact the liquid supply position or disposed near the position to jet the liquid. No member is disposed in front of the liquid jetting passage grooves 121, so that the liquid jetted from the liquid jetting passage grooves 121 reliably concentrates on a target position without scattering around. Therefore, efficient washing and replenishment are possible with a small amount of liquid.

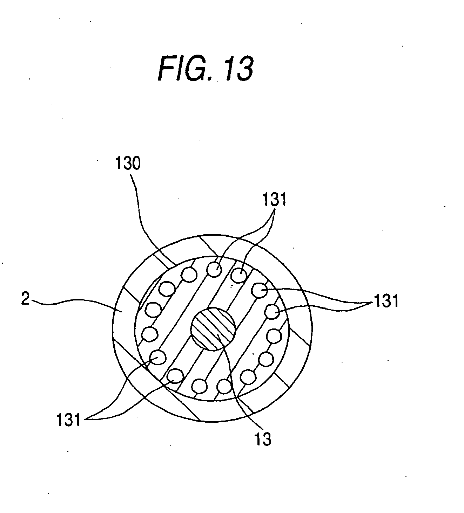

As described above, inplace of the liquid feedpassage grooves 122 formed in the outer circumferential surface of the stopper member 116, as shown in Fig. 13, liquid feed passage holes 131 formed by through holes can be formed in the stopper member 130. The liquid feed passage holes 131 are plurally formed circumferentially, and their circumferential pitches are set equal to or less than the circumferential widths of the liquid jetting passage grooves 121 provided in the partition member 114, whereby at least a part of the liquid feed passage holes 131 communicates with the liquid jetting passage grooves 121 regardless of the rotating position of the stopper member 130.

## Claims

1. A high frequency treatment tool (1,100) which can be inserted into a body cavity via a treatment tool insertion channel (c) of an endoscope, the high frequency treatment tool (1,100) comprising:
a flexible sheath (2),
a treatment tool main body (10) comprising a flexible cord (11) and a straight electrode member (13) on a tip end of the flexible cord (11), the treatment tool main body (10) being attached inside the flexible sheath (2), and a high frequency current capable of being applied to the straight electrode member (13);
a partition member (114) comprising an insertion hole (115) which the electrode member (13) is stuck out from and withdrawn into, the partition member (114) being fixedly attached inside the flexible sheath (2) in such a manner that an tip face of the partition member (114) is provided at almost the same position as a tip end face of the flexible sheath (2); and
a stopper member (116) that restricts a sticking-out length of the electrode member (13) from the insertion hole (115), the stopper member (116) being formed in the treatment tool main body (10) so as to come into contact with and separate from a base end side surface (114a) of the partition member (114),
wherein at least one liquid jetting passage (121) for jetting a liquid supplied from inside of the flexible sheath (2) is formed in the partition member (114), and
a liquid feed passage (22) is formed in the stopper member (116) and the liquid feed passage (122) communicates with the liquid jetting passage (121) at an arbitrary rotating position of the stopper member (116).

2. The high frequency treatment tool (1,100) according to claim 1, wherein the liquid jetting passage (121) comprises a plurality of grooves (121) or through holes provided at an outer circumferences of the partition member (114), and the liquid feed passage (122) comprises a plurality of grooves (122) or through holes provided at an outer circumferences of the stopper member (116).

## Patentansprüche

1. Hochfrequenz-Behandlungsinstrument (1,100), das über einen Behandlungsinstrument-Einführungskanal (C) eines Endoskops in einen Körperhohlraum eingeführt werden kann, wobei das Hochfrequenz-Behandlungsinstrument (1,100) umfasst:
einen flexiblen Schaft (2);
einen Behandlungsinstrument-Hauptkörper (10), umfassend ein flexibles Kabel (11) und ein gerades Elektrodenglied (13) an einem Spitzenende des flexiblen Kabels (11), wobei der Behandlungsinstrument-Hauptkörper (10) in dem flexiblen Schaft (2) befestigt ist und ein Hochfrequenzstrom an das gerade Elektrodenglied (13) angelegt werden kann;
ein Teilungsglied (114), umfassend ein Einführungsloch (115), aus dem das Elektrodenglied (13) heraussteht und in das das Elektrodenglied (13) zurückgezogen wird, wobei das Teilungsglied (114) starr innerhalb des flexiblen Schafts (2) derart befestigt ist, dass eine Spitzenfläche des Teilungsglieds (114) bei nahezu derselben Position wie eine Spitzenendfläche des flexiblen Schafts (2) bereitgestellt ist; und
ein Stoppglied (116), das eine Herausstehlänge des Elektrodenglieds (13) aus dem Einführungsloch (115) begrenzt, wobei das Stoppglied (116) in dem Behandlungsinstrument-Hauptkörper (10) ausgebildet ist, um mit einer Basisendseitenoberfläche (114a) des Teilungsglieds (114) in Kontakt zu kommen und sich von dieser zu trennen,
wobei zumindest ein Flüssigkeitsausströmungsdurchlass (121) zum Ausströmen einer Flüssigkeit, die von innerhalb des flexiblen Schafts (2) bereitgestelltwird, in dem Teilungsglied (114) ausgebildet ist, und
ein Flüssigkeitszufuhrdurchlass (122) in dem Stoppglied (116) ausgebildet ist und der Flüssigkeitszufuhrdurchlass (122) mit dem Flüssigkeitsausströmungsdurchlass (121) bei einer beliebigen Drehposition des Stoppglieds (116) kommuniziert.

2. Hochfrequenz-Behandlungsinstrument (1,100) nach Anspruch 1, wobei der Flüssigkeitsausströmungsdurchlass (121) eine Mehrzahl an Vertiefungen (121) oder Durchgangslöcher umfasst, die auf einem äußeren Umfang des Teilungsglieds (114) bereitgestellt sind, und der Flüssigkeitszufuhrdurchlass (122) eine Mehrzahl an Vertiefungen (122) oder Durchgangslöcher umfasst, die auf einem äußeren Umfang des Stoppglieds (116) bereitgestellt sind.

## Revendications

1. Outil de traitement à haute fréquence (1, 100) qui peut être inséré dans une cavité corporelle via un canal d'insertion (C) pour outil de traitement dans un endoscope, l'outil de traitement à haute fréquence (1, 100) comprenant :
une gaine flexible (2) ;
un corps principal d'outil (10) comprenant un câble flexible (11) et un élément formant électrode droite (13) sur une extrémité terminale du câble flexible (11), le corps principal (10) de l'outil de traitement étant attaché à l'intérieur de la gaine flexible (2), et un courant à haute fréquence est capable d'être appliqué à l'élément formant électrode droite (13) ;
un élément formant cloison (114) comprenant un trou d'insertion (115)
dans lequel l'élément formant électrode (13) est enfiché ou hors duquel celui-ci peut être extrait, l'élément formant cloison (114) étant attaché de manière fixe à l'intérieur de la gaine flexible (2) de telle manière qu'une face terminale de l'élément formant cloison (114) est prévue pratiquement à la même position qu'une face d'extrémité terminale de la gaine flexible (2) ; et
un élément d'arrêt (116) qui restreint une longueur d'extraction de l'élément formant électrode (13) hors du trou d'insertion (115), l'élément d'arrêt (116) étant formé dans le corps principal (10) de l'outil de traitement de manière à venir en contact avec et à se séparer depuis une surface latérale (114a) à l'extrémité de base de l'élément formant cloison (114),
dans lequel au moins un passage de projection de liquide (121) pour projeter un liquide fourni depuis l'intérieur de la gaine flexible (2) est formé dans l'élément formant cloison (114), et
un passage d'alimentation de liquide (122) est formé dans l'élément d'arrêt (116) et le passage d'alimentation de liquide (122) communique avec le passage de projection de liquide (121) à une position de rotation arbitraire de l'élément d'arrêt (116).

2. Outil de traitement à haute fréquence (1, 100) selon la revendication 1, dans lequel le passage de projection de liquide (121) comprend une pluralité de gorges (121) ou de trous traversants prévu(e)s au niveau d'une circonférence extérieure de l'élément formant cloison (114), et le passage d'alimentation de liquide (122) comprend une pluralité de gorges (122) ou de trous traversants prévu(e)s au niveau d'une circonférence extérieure de l'élément d'arrêt (116).
